# EUROPÄISCHE PATENTSCHRIFT

(11) **EP 1 827 541 B1**
(45) Veröffentlichungstag und Bekanntmachung des Hinweises auf die Patenterteilung: **29.05.2013**
(21) Anmeldenummer: 05801717.9
(22) Anmeldetag: 17.10.2005
(51) Int. Cl.: A61M 15/00

(54) **BLISTER FÜR INHALATOREN**
BLISTER FOR INHALERS
BLISTERS POUR INHALATEURS

(30) Priorität: 21.10.2004 EP 04025038
(43) Veröffentlichungstag der Anmeldung: 05.09.2007
(73) Patentinhaber: Boehringer Ingelheim International GmbH, 55216 Ingelheim (DE); Boehringer Ingelheim Pharma GmbH & Co. KG, 55216 Ingelheim (DE)
(72) Erfinder: KRUEGER, Michael, 55218 INGELHEIM (DE); METZGER, Burkhard, 55218 INGELHEIM (DE); TRUNK, Michael, 55218 INGELHEIM (DE); SCHIEWE, Joerg, 55129 MAINZ (DE)
(74) Vertreter: Simon, Elke Anna Maria
(86) Internationale Anmeldenummer: PCT/EP2005/055313
(87) Internationale Veröffentlichungsnummer: WO 2006/045715

(56) Entgegenhaltungen:
- WO-A-01/72605
- WO-A-89/01348
- WO-A-03/055547
- US-A- 5 746 227

## Beschreibung

Die Erfindung betrifft eine Primärverpackung für Inhalationsformulierungen als integraler Bestandteil eines einsatzfähigen Pulverinhalators, insbesondere zur Aufbewahrung von Arzneimittelformulierungen, die Tiotropiumbromid-Monohydrat enthalten.

### Stand der Technik

Die medizinische auf die pulmonale Inhalation ausgerichtete Aerosoltherapie mittels Vernebler, Dosieraerosol oder Trockenpulverinhalator spielt eine wichtige Rolle in der Behandlung von zahlreichen Lungenkrankheiten.

Auf dem Gebiet der Pulverinhalatoren sind Einzeldosen und Mehrdosen-Geräte bekannt. In Einzeldosen-Pulverinhalatoren kann die Dosierung in Form von Kapseln vorgenommen werden, die eine Pulverformulierung enthalten. Pulverformulierungen enthalten den Wirkstoff in mikronisierter Form (mit einer Teilchengröße von ca. 1 - 5 *µ*m), und meist ein oder mehrere Hilfsstoffe. Wird eine Kapsel als Behältnis verwendet, dann wird diese in dem Pulverinhalatoren vor dem Inhalationsmanöver durch Anstechen, Quetschen oder Scheren geöffnet, damit das Pulver durch den Atemzug des Patienten aus der Kapsel gefördert werden kann und ein luftgetragenes Aerosol erzeugt wird, das der Patient einatmet. Bei den Pulverinhalatoren unterscheidet man zwischen Mehrdosis-Pulverinhalatoren, die die Formulierung in Form eines Pulvervorrats enthalten, aus dem durch eine eingebaute Dosiereinheit die jeweilige Einzeldosis entnommen wird und Pulverinhalatoren mit vordosierten, verpackten Einzeldosen, die entweder gemeinsam in dem Gerät bevorratet sind oder bei Gebrauch einzeln in das Gerät eingesetzt werden.

Im Rahmen der vorliegenden Erfindung sind besonders Mehrdosispulverinhalatoren mit vordosierten, verpackten Einzeldosen, die in einem Blisterband oder einer Blisterscheibe angeordnet sind, von Interesse. In jedem Fall ist oft die Art und Weise, wie die Pulverformulierung in dem Gerät verpackt vorliegen, für die Produktqualität und damit die Eignung für die inhalative Anwendungen entscheidend.

Beispiele für Inhalatoren, die auf den genannten beiden Prinzipien aufbauen sind im Stand der Technik bekannt. So offenbaren die DE 3348370 und die DE 3336486 Inhalatoren, die eine scheibenförmige Blisterpackung, die mehrere kreisförmig angeordnete Kavitäten aufweist, beinhalten. Die einzelnen Kavitäten enthalten jeweils eine Dosis eines zur Inhalation bestimmten MedikamentenPulvers. Die Kavitäten sind beidseitig z.B. durch eine Siegelfolie geschlossen. Zum Ausbringen des Medikamentenpulvers wird die Kavität geöffnet. Ein Luftkanal verbindet die geöffneten Kavität mit dem Mundstück des Inhalators. Beispielhaft wird der Inhalator der DE 3336486 näher beschrieben. Dieser weist ein Gehäuse auf, in dem sich eine Kammer (Vorratskammer) findet, die einen Lufteinlass aufweist und in welcher sich ein scheibenförmiger, runder Blister mit abgepackten Medikamententaschen befindet. Der Blister ist lose mit einer runden, rotierbaren Scheibe verbunden. Auf der Scheibe sind umlaufend Löcher ausgebildet, die in axialer Richtung Kontakt mit den Medikamententaschen haben, d.h. die Taschen und Löcher liegen über- bzw. untereinander. Die Kammer weist einen Luftauslass auf. Der Inhalator weist auch einen Kolben auf, der so angeordnet ist, dass er jeweils eine Medikamententasche durchstossend öffnen kann, so dass das Medikament in die Kammer freigesetzt wird und über ein Mundstück eingeatmet werden kann. Es wird auf die Zeichnungen der Patentanmeldung bzw. der US-Patentschrift verwiesen.

Die DE 4106379 beschreibt ein Inhalationsgerät, in das ein Blister oder ähnliches für ein pulverförmiges Medikament eingebracht werden kann. Der Blister besteht aus zwei voneinander abziehbaren Materialbahnen, die wenigstens einen Behälter definieren, in dem sich das Medikament befindet. Das Gerät ist versehen mit einer Einrichtung, die zum Öffnen eines Behälters die beiden Materialbahnen an einer Öffnungsstation voneinander abzieht. Über ein Auslassteil, etwa ein Mundstück, das mit dem geöffneten Behälter verbunden ist, kann der Benutzer das pulverförmige Medikament aus dem geöffneten Behälter inhalieren. Dabei kann eine der Materialbahnen auch eine Trägerbahn mit mehreren Taschen sein und die andere Materialbahn eine Abdeckbahn. Jede Tasche und der angrenzende Bereich der Abdeckbahn bilden dann einen Behälter. An der Öffnungsstation kann eine Antriebseinrichtung vorgesehen sein, die die Trägerbahn und die Abdeckbahn voneinander abzieht. Diese Antriebseinrichtung besteht z.B. aus zwei Antriebsrädern (z.B. Zahnräder), welche die Abdeckbahn in Antriebseingriff zwischen sich halten. Auch in diesem Fall definiert jeder einzelne Blister ein Art Vorratskammer im Inhalator, die über einen Luftkanal mit dem Mundstück verbunden ist.

Bezüglich der Verpackung der Medikamentenpulver unterscheidet man zwischen der Primärverpackung und der Sekundärverpackung.
Die Primärverpackung ist dadurch gekennzeichnet, dass sie sich unmittelbar mit der Inhalationsformulierung in Kontakt befindet.
Gegebenenfalls kann die Primärverpackung von einem zweiten äußeren Schutz umgeben sein, dem Sekundärpackmittel.
Das Primärpackmittel kann dabei z.B. eine Kapsel, ein fester oder flexibler Blister mit Kavitäten oder eine Scheibe mit Kavitäten sein. Im Rahmen der vorliegenden Erfindung sind Kapseln jedoch ausgenommen.

Das Sekundärpackmittel kann ein Blister sein, eine Tasche, ein Beutel oder ein anderes Behältnis. Das Sekundärpackmittel umschließt dabei in der Regel das Primärpackmittel vollständig. Sekundärpackmittel werden insbesondere dann verwendet, wenn das Primärpackmittel keinen ausreichenden Schutz vor Feuchtigkeit bietet.

Die Primärverpackungen können aus synthetischen Kunststoffen, wie Polyethylen, Polycarbonat, Polyester, Polypropylen oder Polyethylenterephthalat u.a. gefertigt sein. Auch eignen sich zum Beispiel Verbundmaterialien aus Kunststoff und Aluminium oder nur Aluminium.

Das Primärpackmittel und gegebenenfalls das Sekundärpackmittel haben die Aufgabe, den Wirkstoff als auch die gesamte Inhalationsformulierung vor chemischer oder physikalischer Veränderung zu schützen. Zu den physikalischen Veränderungen zählen dabei insbesondere Veränderungen, die das Ausbringen der vorbestimmten Feinpartikeldosis verändern können. Unter dem Begriff Feinpartikeldosis versteht man dabei die Dosis, die die Lunge des Patienten erreicht. Letztere wird von den Wechselwirkungen der mikronisierten Wirkstoffpartikel untereinander als auch der Wechselwirkungen mit den Hilfsstoffen beeinflusst. Es hat sich gezeigt, dass besonders durch Änderung des Feuchtigkeitsgrades im Inneren der Verpackung diese Wechselwirkungen derart verändert werden können, dass die Feinpartikeldosis deutlich verändert, insbesondere deutlich vermindert wird. Derartige Veränderungen schließen dabei das Eindringen von Wasser in die Verpackung genauso ein, wie das Entfernen von Wasser aus dem Inneren der Verpackung.

Daher ist es eine Hauptaufgaben der Verpackung, die chemische Zusammensetzung der Atmosphäre im Inneren der Verpackung konstant zu halten, um physikalischen oder chemischen Veränderungen der Wirkstoffformulierung vorzubeugen, bzw. die Inhalationsformulierung stabil zu halten. In diesem Zusammenhang unterscheidet man zwischen einer auf eine kurze Zeit ausgerichteten Stabilität, die die Inhalationsformulierung *per se* besitzen muss, auch wenn sich nicht durch das Packmittel ausreichend geschützt wird ("*in-use-Stabilität')* und der Langzeitstabilität, d.h. der Stabilität, die gewährleistet sein muss, solange sich die Inhalationsformulierung in dem ungeöffneten Packmittel befindet.

Die Verpackung muss also gewährleisten, dass die Inhalationsformulierung langfristig stabil bleibt. Kann das Material und die konstruktive Gestaltung des Primärpackmittels dies nicht gewährleisten, bedarf es des Sekundärpackmittels. Die Auswahl eines geeigneten Materials für das Primärpackmittel wird durch zwei Faktoren bestimmt: Zum einen muss das Material die angesprochene Schutzfunktion erfüllen können. Zum anderen muss das Material derart sein, dass der Primärpackung die zur Verwendung in dem Pulverinhalator notwendige Form gegeben werden kann und das Primärpackmittel die ihr zugedachte Funktion erfüllen kann.
Bevorzugt werden als Materialien Kunststoffe aus der Gruppe der thermoplastische Polymere wie z.B. Polystyrole, Polyolefine, Polyamide, Polyvinylchloride oder Polyurethane verwendet. Diese besitzen die notwendige Steifheit bzw. Beweglichkeit, um die mechanischen Aufgaben des Primärpackmittels zu erfüllen. Ihr Nachteil besteht darin, dass sie in beide Richtungen für Luftfeuchtigkeit durchlässig sind. Damit besteht ein Bedarf, die Fähigkeit der Verpackungen, das Inhalationspulver stabil aufzubewahren, zu erhöhen.

Dokument WO03/055547 offenbart einen Blister für einen Pulverinhalator, in der Form eines Blisterstreifen, bestehend aus einem Basiselement mit einer Mehrzahl nach einer Seite hin offenen Kavitäten, die durch Stege voneinander getrennt sind und die eine Inhalationsformulierung enthalten, wobei die Öffnungen der Kavitäten versiegelt sind und die Wandung der Kavitäten aus zwei Schichten, einer inneren und einer darüber liegenden äußeren Schicht besteht. Die innere Schicht bildet die unmittelbare Wandung der Kavitäten und steht im Kontakt mit der Inhalationsformulierung. Die beiden Schichten bestehen aus Polymerzusammensetzungen und die innere Schicht ist mit einem entwässerndem Material versehen.

Es wurde nun überraschenderweise beobachtet, dass pharmazeutische Formulierungen, die Tiotropiumbromid-Monohydrat als Wirkstoff enthalten in ihren physico-chemischen Eigenschaften auf Änderung des Feuchtigkeitsgrades reagieren. Eine Lösung dieses Problems konnte dadurch gefunden werden, dass als Primärpackmittel Materialien verwendet wurden, in die ein Entwässerungsmittel eingearbeitet ist.

### Beschreibung der Erfindung

Die vorliegende Erfindung betrifft daher Verpackungen in Form eines Blisterbands oder einer Blisterscheibe für Inhalationspulver enthaltend einen feuchtigkeitsempfindlichen Arzneistoff oder eine feuchtigkeitsempfindliche Formulierung zur Verwendung als integraler Bestandteil eines einsatzfähigen Pulverinhalators, um Arzneistoff oder Formulierung vor einer Feuchtigkeitsaufnahme (z. B. einer Wasseraufnahme) zu schützen. Eine weitere Aufgabe der Verpackung besteht darin, den Austausch von Materie zwischen ihrem Inneren und der Umgebung zu minimieren.

### Beschreibung der Erfindung im Detail

Die Erfindung betrifft einen Blister ausgewählt aus der Gruppe der Blisterstreifen oder der Blisterscheiben, zur Verpackung für Inhalationsformulierungen enthaltend einen feuchtigkeitsempfindlichen Arzneistoff oder eine feuchtigkeitsempfindliche Arzneistoffformulierung, wobei der Blister zunächst ein Basiselement aufweist, welches aus einem thermoplastischen Kunststoff besteht und wenigstens zwei durch einen Steg von einander getrennte Kavitäten aufweist. Die Kavitäten sind wenigstens nach einer Seite hin offen, gegebenenfalls auch nach zwei, sich gegenüberliegenden Seiten. Diese Öffnungen sind im einsatzfertigen Blister geschlossen, z.B. durch eine mit dem Basiselement fest verbundene Siegelfolie. Bevorzugt sind Blisterscheiben.

In den Kavitäten befindet sich das Inhalationspulver. Der befüllte Blister mit verschlossenen Kavitäten ist integraler Bestandteil eines gebrauchsfertigen Pulverinhalators.

Die Wandung der Kavität bestent aus wenigstens zwei Schichten, einer inneren und wenigstens einer darüber liegenden äußeren Schicht. Dabei bildet die innere Schicht die unmittelbare Wandung der Kavität und steht damit in Kontakt mit der Inhalationsformulierung. In diesem Fall besteht die innere Schicht aus der Polymerzusammensetzung ohne Entwässerungsmittel. Die diese umgebende zweite Schicht, die die Inhalationsformulierung nicht berührt, besteht aus der Polymerzusammensetzung mit Entwässerungsmittel. Dabei können die Polymere der inneren und der wenigstens einen zweiten Schicht so gewählt werden, dass sie nicht stoffschlüssig miteinander verbunden werden oder dass sie stoffschlüssig miteinander verbunden werden.

Die Gestalt des erfindungsgemäßen Blisters wird durch den zu verwendenden Pulverinhalator vorgegeben. Das Basiselement kann so ausgestaltet sein, dass die pharmazeutische Formulierung mit ihm direkt in Kontakt steht oder indirekt.

Mit dem erfindungsgemäßen Blister wird eine Inhalationsformulierung, die bevorzugt ein feuchtigkeitsempfindliches Anticholinergikum beinhaltet, gegebenenfalls in einem amorphen Zustand vorliegt, und für längere Zeit in einem Pulverinhalator aufbewahrt werden muss, bevor sie ausgebracht wird, gegenüber dem Eindringen von Feuchtigkeit von der Außenumgebung besser abgeschirmt als es bei den aus dem Stand der Technik bekannten, vergleichbaren Blistern der Fall ist.

Das verminderte Eindringen von Feuchtigkeit in den Blister verbessert die Langzeit- und die Anbruchstabilität einer Inhalationsformulierung.

Die Langzeitstabilität wird bei einem verpackten und versiegelten Blister oder einem verpackten und versiegelten Pulverinhalator durch Langzeit-Lagerungen gemessen, in dem man die Verlängerung des Feuchteschutzes in Abhängigkeit von der Blister- bzw. Pulverinhalator-Leckrate in mg/Jahr vom Masseanteil Molekularsieb darstellt.

Die Anbruchstabilität wird bei einem nicht mehr verpackten und versiegelten (d.h. angebrochenen) Pulverinhalator oder der aus einer Blistereinheit entnommenen Kapsel gemessen. Die Parameter der Messung entsprechen den Angaben, wie für die Messung der Langzeitstabilität dargestellt.

Die Langzeit- und Anbruchstabilität wird beispielsweise bei 30 - 40°C und einer Feuchtigkeit von 75% in mit Inhalationspulver gefüllten und ungefüllten Blister-Packungen bzw. Pulverinhalatoren gemessen.

Erfindungsgemäß ist es nicht notwendig, aber bevorzugt, dass alle Wände der Kavität aus dem gleichen Material bestehen. Bei einer Kavität kann zumindest die die Öffnung verschließende Wandung von anderem Material als die restlichen Wandungen sein.

Inhalationsformulierung steht hierbei bevorzugt für eine pharmazeutische Pulverformulierung, die als aktiven Bestandteil ein Anticholinergikum enthält und deren Partikel eine Größe von weniger als 100 Mikrometern aufweisen.

"Integraler Bestandteil eines einsatzfähigen Inhalators" bedeutet, dass der Blister ein Element in dem Inhalator ist, ohne das die Beschickung des Inhalators mit der Arzneimittelformulierung (Inhalationsformulierung) zum Zweck der Inhalation nicht möglich ist bzw. nicht vorgesehen ist. Dieses Element kann in gebrauchsfertigem Zustand (einsatzfähigen Zustand) fest mit dem Inhalator verbunden sein, so dass sie nicht zerstörungsfrei oder ohne den Inhalator zu beschädigen entnommen werden kann, oder es ist zerstörungsfrei lose bzw. lösbar mit dem Inhalator verbunden.
Einsatzfähig bedeutet, dass das erfindungsgemäße Behältnis in den Inhalator eingesetzt vorliegt. Gegebenenfalls wird das Behältnis mechanisch durch Bauteile des Inhalators geöffnet und/oder in dem Inhalator zum Ort der Ausbringung transportiert. Die Reihenfolge dieser beiden Schritte kann auch verändert werden.

Bei dem Material für das Basiselement handelt es sich um Polymerzusammensetzungen, die wenigstens ein thermoplastisches Polymer, wenigstens ein entwässerndes Mittel und optional wenigstens ein Elastomer und/oder ggf. Weichmacher und/oder weitere Fasern enthalten. Das Material enthält dabei weder Gelatine, noch Cellulose oder Stärke oder Derivate derselben.

Bevorzugte Polymerzusammensetzungen bestehen beispielsweise aus
- 50 - 80 Gew.% eines oder mehrerer thermoplastischer Polymere,
- b) 20 - 50 Gew.% eines oder mehrerer Entwässerungsmittel,
- 2 - 8 Gew.% eines oder mehrerer Elastomere
- gegebenenfalls 1 bis 4 Gew.% einer synthetischen und/oder pflanzlichen und/oder tierischen Faser der Länge 0,5 bis 4 mm.

Bevorzugt liegt die Menge des Entwässerungsmittels bei unter 30 Gew.%, stärker bevorzugt bei bis zu 25 Gew.%.

Als Polymerkomponente des Kunststoffes kommen vor allem thermoplastische Polymere in Frage wie z.B. Polystyrole, Polyolefine, Polyamide, Polyvinylchloride oder Polyurethane. Besonders bevorzugt sind Polyethylen insbesondere Polyethylen mit einer Dichte zwischen 900 und 1000 kg/m³, bevorzugt von 940 - 980 kg/m³ , besonders bevorzugt von 960 kg/m³ (high-density Polyethylen), Polycarbonat, Polyester, Polypropylen oder Polyethylenterephthalat.

Als entwässernde Mittel kommen beispielsweise Kieselgele, Aktivkohle, Zeolithe, Aluminiumoxid, Magnesiumsulfat, Molekularsiebe u.ä. in Frage.

Als Elastomere können z.B. ein oder mehrere Substanzen enthalten sein aus der Gruppe der Styrol-Butadien-Kautschuke (SBR), Styrol-Ethylen-Butylen-StyrolCopolymere (SEBS), Butyl-Kautschuke, Ethylen-Propylen-Kautschuke (EPR), Ethylen-Propylen-Dien-Kautschuke (EPDM), Ethylen-Vinylacetat-Copolymere (EVA), Ethylen-Acrylat-Copolymere, Acrylnitril-Butadien-Copolymere, Polynorbonene, Polyiisoprene, Polychloroprene und Polybutadiene beschrieben werden.

Schließlich kann die Polymerzusammensetzung auch weitere anorganische oder organische Zusätze enthalten, die die folgende Funktion haben: Weichmacher, Stabilisator, Farbstoff, Pigment oder ähnliche.

Bevorzugt werden entwässernde Kunststoffe - also Kunststoffe, die ein Entwässerungsmittel enthalten - verwendet, die spritz- oder blasgusstechnisch verarbeitet werden können. Bevorzugt sind zudem Kunststoffe für deren Verarbeitung kein Formtrennmittel notwendig ist, das ein Anhaften des Füllguts an der Wand bewirken kann. Das hat den Vorteil, dass das Innere des Behältnisses nicht vom Formtrennmittel gereinigt werden muss, um z.B. den amtlichen Bestimmungen (z.B. nach DAB (Deutsches Arzneibuch)) genüge zu tun, die die Verwendung von Formtrennmitteln für Primärpackmittel einschränken.

In einer bevorzugten Ausführungsform besitzt der entwässernde Kunststoff keine ausgeprägte Adhäsion für pharmazeutisch-chemische Stoffe, insbesondere für Partikel mit lungengängiger Größe, so dass bei Verwendung des Blisters in einem Inhalator der gesamte Inhalt der Kavität freigesetzt werden kann. Dies gewährleistet eine exaktere Dosierung, insbesondere des lungengängigen Feinanteils der pharmazeutischen Zubereitung.

Weitere Angaben zur Zusammensetzung oder die Verarbeitung betreffend können dem Stand der Technik entnommen werden, insbesondere der EP599690, EP432438 oder der EP400460.

In einer Ausführungsform kann die Blisterwandung Regionen mit unterschiedlicher Zusammensetzung aus Polymer/ Entwässerungsmittel enthalten. Auf diese Art ist es möglich, die Bereiche des Behälters, an denen die Inhalator-eigenen Mittel zur Öffnung der Kavität angreifen aus einem anderen Material zu gestalten, als die restliche Wandung. Auch können die Entwässerungsmittel nur in Bereichen eingesetzt werden, die nicht Inhalationsformulierung-berührend sind, usw..

In wieder einer anderen Ausführungsform, wird das Basiselement aus einem Material ohne Entwässerungsmittel gefertigt, auf das nach Befüllung und Verschließung der Kavitäten eine Schicht aus Polymerzusammensetzung mit Entwässerungsmittel aufgebracht wird.

Der erfindungsgemäße Blister bietet vor allem dann Vorteile, wenn Wirkstoffe, Hilfsstoffe oder Formulierungen besonders vor einer Wasseraufnahme geschützt werden müssen. Beispielsweise trifft das auf Inhalationspulver zu, die mittels Sprühtrocknung hergestellt wurden und/oder für Wirkstoffe, Hilfsstoffe und Formulierungen, die im amorphen Zustand vorliegen.

Bevorzugt werden inhalativ wirksame Wirkstoffe eingesetzt.

Besonders bevorzugt sind in diesem Zusammenhang Arzneimittel, die ausgewählt sind aus der Gruppe bestehend aus Anticholinergika, Betamimetika, Steroiden, Phosphodiesterase IV-inhibitoren, LTD4-Antagonisten und EGFR-Kinase-Hemmer, Triptane, CGRP-Antagonisten, Phosphodiesterase-V-Inhibitoren, sowie Kombinationen von zwei oder mehreren solchen Wirkstoffen, z.B. einem Betamimetikum plus einem Anticholinergikum oder die Kombination von zwei oder mehr Arzneimitteln aus derselben Gruppe, z.B. die Kombination von zwei oder mehr Anticholinergika.

Im einzelnen seien als Beispiele für die wirksamen Bestandteile oder deren Salze genannt:

Zur Anwendung gelangende Anticholinergika sind bevorzugt ausgewählt aus der Gruppe bestehend aus Tiotropium bzw. Tiotropiumbromid, Oxitropiumbromid, Flutropiumbromid, Ipratropiumbromid, Glycopyrroniumsalze, Trospiumchlorid, Tolterodin, 2,2-Diphenylpropionsäuretropenolester-methobromid, 2,2-Diphenylpropionsäurescopinester-methobromid, 2-Fluor-2,2-Diphenylessigsäurescopinestermethobromid, 2-Fluor-2,2-Diphenylessigsäuretropenolester-methobromid, 3,3',4,4'-Tetrafluorbenzilsäuretropenolester-Methobromid, 3,3',4,4'-Tetrafluorbenzilsäurescopinester-Methobromid, 4,4'-Difluorbenzilsäuretropenolester-Methobromid, 4,4'-Difluorbenzilsäurescopinester-Methobromid, 3,3'-Difluorbenzilsäuretropenolester-Methobromid, 3,3'-Difluorbenzilsäurescopinester-Methobromid, 9-Hydroxy-fluoren-9-carbonsäuretropenolester -Methobromid, 9-Fluor-fluoren-9-carbonsäuretropenolester -Methobromid, 9-Hydroxy-fluoren-9-carbonsäurescopinester -Methobromid, 9-Fluor-fluoren-9-carbonsäurescopinester Methobromid, 9-Methyl-fluoren-9-carbonsäuretropenolester Methobromid, 9-Methylfluoren-9-carbonsäurescopinester Methobromid, Benzilsäurecyclopropyltropinester-Methobromid, 2,2-Diphenylpropionsäurecyclopropyltropinester -Methobromid, 9-Hydroxy-xanthen-9-carbonsäurecyclopropyltropinester-Methobromid, 9-Methyl-fluoren-9-carbonsäurecyclopropyltropinester-Methobromid, 9-Methyl-xanthen-9-carbonsäurecyclopropyltropinester-Methobromid, 9-Hydroxy-fluoren-9-carbonsäurecyclopropyltropinester-Methobromid, 4,4'-Difluorbenzilsäuremethylestercyclopropyltropinester -Methobromid, 9-Hydroxy-xanthen-9-carbonsäuretropenolester -Methobromid, 9-Hydroxy-xanthen-9-carbonsäurescopinester Methobromid, 9-Methyl-xanthen-9-carbonsäuretropenolester -Methobromid, 9-Methyl-xanthen-9-carbonsäure-scopinester -Methobromid, 9-Ethyl-xanthen-9-carbonsäuretropenolester Methobromid, 9-Difluormethyl-xanthen-9-carbonsäuretropenolester -Methobromid und 9-Hydroxymethyl-xanthen-9-carbonsäurescopinester -Methobromid, gegebenenfalls in Form ihrer Racemate, Enantiomere oder Diastereomere und gegebenenfalls in Form ihrer Säureadditionssalze, Solvate und/oder Hydrate.

Zur Anwendung gelangende Betamimetika sind bevorzugt ausgewählt aus der Gruppe bestehend aus Albuterol, Bambuterol, Bitolterol, Broxaterol, Carbuterol, Clenbuterol, Fenoterol, Formoterol, Hexoprenaline, Ibuterol, Indacaterol, Isoetharine, Isoprenaline, Levosalbutamol, Mabuterol, Meluadrine, Metaproterenol, Orciprenaline, Pirbuterol, Procaterol, Reproterol, Rimiterol, Ritodrine, Salmeterol, Salmefamol, Soterenot, Sulphonterol, Tiaramide, Terbutaline, Tolubuterol, CHF-1035, HOKU-81, KUL-1248, 3-(4-{6-[2-Hydroxy-2-(4-hydroxy-3-hydroxymethyl-phenyl)-ethylamino]-hexyloxy}-butyl)-benzolsulfonamid, 5-[2-(5,6-Diethyl-indan-2-ylamino)-1-hydroxy-ethyl]-8-hydroxy-1*H*-quinolin-2-on, 4-hydroxy-7-[2-{[2-{[3-(2-phenylethoxy)propyl]sulphonyl}ethyl]-amino}ethyl]-2(3H)-benzothiazolon, 1-(2-Fluoro-4-hydroxyphenyl)-2-[4-(1-benzimidazolyl)-2-methyl-2-butylamino]ethanol , 1-[3-(4-methoxybenzyl-amino)-4-hydroxyphenyl]-2-[4-(1-benzimidazolyl)-2-methyl-2-butylamino]ethanol, 1-[2H-5-hydroxy-3-oxo-4H-1,4-benzoxazin-8-yl]-2-[3-(4-N,N-dimethylaminophenyl)-2-methyl-2-propylamino]ethanol , 1-[2H-5-hydroxy-3-oxo-4H-1,4-benzoxazin-8-yl]-2-[3-(4-methoxyphenyl)-2-methyl-2-propylamino]ethanol , 1-[2H-5-hydroxy-3-oxo-4H-1,4-benzoxazin-8-yl]-2-[3-(4-n-butyloxyphenyl)-2-methyl-2-propylamino]ethanol, 1-[2H-5-hydroxy-3-oxo-4H-1,4-benzoxazin-8-yl]-2-{4-[3-(4-methoxyphenyl)-1,2,4-triazol-3-yl]-2-methyl-2-butylamino}ethanol , 5-hydroxy-8-(1-hydroxy-2-isopropylaminobutyl)-2H-1,4-benzoxazin-3-(4H)-on, 1-(4-amino-3-chloro-5-trifluormethylphenyl)-2-tert.-butylamino)ethanol und 1-(4-ethoxycarbonylamino-3-cyano-5-fluorophenyl)-2-(tert.-butylamino)ethanol, gegebenenfalls in Form ihrer Racemate, Enantiomere oder Diastereomere und gegebenenfalls in Form ihrer pharmakologisch verträglichen Säureadditionssalze, Solvate und/oder Hydrate.

Zur Anwendung gelangende Steroide sind bevorzugt ausgewählt aus der Gruppe bestehend aus Prednisolon, Prednison, Butixocortpropionat, RPR-106541, Flunisolid, Beclomethason, Triamcinolon, Budesonid, Fluticason, Mometason, Ciclesonid, Rofleponid, ST-126, Dexamethason, 6α,9α-Difluoro-17α-[(2-furanylcarbonyl)oxy]-11β-hydroxy-16α-methyl-3-oxo-androsta-1,4-dien-17β-carbothionsäure (S)-fluoromethylester, 6α,9α-Difluoro-11β-hydroxy-16α-methyl-3-oxo-17α-propionyloxy-androsta-1,4-dien-17β-carbothionsäure (S)-(2-oxo-tetrahydro-furan-3S-yl)ester und Etiprednol-dichloroacetat (BNP-166), gegebenenfalls in Form ihrer Racemate, Enantiomere oder Diastereomere und gegebenenfalls in Form ihrer Salze und Derivate, ihrer Solvate und/oder Hydrate.

Zur Anwendung gelangende PDE IV-inhibitoren sind bevorzugt ausgewählt aus der Gruppe bestehend aus Enprotyllin, Theophyllin, Roflumilast, Ariflo (Cilomilast), CP-325,366, BY343, D-4396 (Sch-351591), AWD-12-281 (GW-842470), N-(3,5-Dichloro-1-oxo-pyridin-4-yl)-4-difluoromethoxy-3-cyclopropylmethoxybenzamid, NCS-613, Pumafentine, (-)p-[(4*a*R*,10*b*S*)-9-Ethoxy-1,2,3,4,4a,10b-hexahydro-8-methoxy-2-methylbenzo[s][1,6]naphthyridin-6-yl]-N,N-diisopropylbenzamid, (R)-(+)-1-(4-Bromobenzyl)-4-[(3-cyclopentyloxy)-4-methoxyphenyl]-2-pyrrolidon, 3-(Cyclopentyloxy-4-methoxyphenyl)-1-(4-N'-[N-2-cyano-S-methyl-isothioureido]benzyl)-2-pyrrolidon, cis[4-Cyano-4-(3-cyclopentyloxy-4-methoxyphenyl)cyclohexan-1-carbonsäure], 2-carbomethoxy-4-cyano-4-(3-cyclopropylmethoxy-4-difluoromethoxyphenyl)cyclohexan-1-on, cis[4-Cyano-4-(3-cyclopropylmethoxy-4-difluoromethoxyphenyl)cyclohexan-1-ol], (R)-(+)-Ethyl[4-(3-cyclopentyloxy-4-methoxyphenyl)pyrrolidin-2-yliden]acetat, (S)-(-)-Ethyl[4-(3-cyclopentyloxy-4-methoxyphenyl)pyrrolidin-2-yliden]acetat, CDP840, Bay-198004, D-4418, PD-168787, T-440, T-2585, Arofyllin, Atizoram, V-11294A, CI-1018, CDC-801, CDC-3052, D-22888, YM-58997, Z-15370, 9-Cyclopentyl-5,6-dihydro-7-ethyl-3-(2-thienyl)-9*H*-pyrazolo[3,4-c]-1,2,4-triazolo[4,3-a]pyridin und 9-Cyclopentyl-5,6-dihydro-7-ethyl-3-(*tert*-butyl)-9*H-*pyrazolo[3,4-c]-1,2,4-triazolo[4,3-a]pyridin, gegebenenfalls in Form ihrer Racemate, Enantiomere oder Diastereomere und gegebenenfalls in Form ihrer pharmakologisch verträglichen Säureadditionssalze, Solvate und/oder Hydrate.

Zur Anwendung gelangende LTD4-Antagonisten sind bevorzugt ausgewählt aus der Gruppe bestehend aus Montelukast, 1-(((R)-(3-(2-(6,7-Difluoro-2-quinolinyl)ethenyl)phenyl)-3-(2-(2-hydroxy-2-propyl)phenyl)thio)methylcyclopropanessigsäure, 1-(((1(R)-3(3-(2-(2,3-Dichlorothieno[3,2-b]pyridin-5-yl)-(E)-ethenyl)phenyl)-3-(2-(1-hydroxy-1-methylethyl)phenyl)propyl)thio)methyl)cyclopropanessigsäure, Pranlukast, Zafirlukast, [2-[[2-(4-tert-Butyl-2-thiazolyl)-5-benzofuranyl]oxymethyl]phenyl]essigsäure, MCC-847 (ZD-3523), MN-001, MEN-91507 (LM-1507), VUF-5078, VUF-K-8707 und L-733321, gegebenenfalls in Form ihrer Racemate, Enantiomere oder Diastereomere, gegebenenfalls in Form ihrer pharmakologisch verträglichen Säureadditionssalze sowie gegebenenfalls in Form ihrer Salze und Derivate, ihrer Solvate und/oder Hydrate.

Zur Anwendung gelangende EGFR-Kinase-Hemmer sind bevorzugt ausgewählt aus der Gruppe bestehend aus Cetuximab, Trastuzumab, ABX-EGF, Mab ICR-62, 4-[(3-Chlor-4-fluorphenyl)amino]-6-{[4-(morpholin-4-yl)-1-oxo-2-buten-1-yl]amino}-7-cyclopropylmethoxy-chinazolin, 4-[(R)-(1-Phenyl-ethyl)amino]-6-{[4-(morpholin-4-yl)-1-oxo-2-buten-1-yl]amino}-7-cyclopentyloxy-chinazolin, 4-[(3-Chlor-4-fluorphenyl)amino]-6-{[4-((R)-6-methyl-2-oxo-morpholin-4-yl)-1-oxo-2-buten-1-yl]amino}-7-[(S)-(tetrahydrofuran-3-yl)oxy]-chinazolin, 4-[(3-Chlor-4-fluor-phenyl)amino]-6-[2-((S)-6-methyl-2-oxo-morpholin-4-yl)-ethoxy]-7-methoxy-chinazolin, 4-[(3-Chlor-4-fluorphenyl)amino]-6-({4-[N-(2-methoxy-ethyl)-N-methyl-amino]-1-oxo-2-buten-1-yl}amino)-7-cyclopropylmethoxy-chinazolin, 4-[(R)-(1-Phenyl-ethyl)amino]-6-({4-[N-(tetrahydropyran-4-yl)-N-methyl-amino]-1-oxo-2-buten-1-yl}amino)-7-cyclopropylmethoxy-chinazolin, 4-[(3-Chlor-4-fluorphenyl)amino]-6-({4-[N-(2-methoxy-ethyl)-N-methyl-amino]-1-oxo-2-buten-1-yl}amino)-7-cyclopentyloxychinazolin, 4-[(3-Chlor-4-fluorphenyl)amino]-6-{[4-(N,N-dimethylamino)-1-oxo-2-buten-1-yl]amino}-7-[(R)-(tetrahydrofuran-2-yl)methoxy]-chinazolin, 4-[(3-Ethinyl-phenyl)amino]-6,7-bis-(2-methoxy-ethoxy)-chinazolin, 4-[(R)-(1-Phenylethyl)amino]-6-(4-hydroxy-phenyl)-7H-pyrrolo[2,3-d]pyrimidin, 3-Cyano-4-[(3-chlor-4-fluorphenyl)amino]-6-{[4-(N,N-dimethylamino)-1-oxo-2-buten-1-yl]amino}-7-ethoxy-chinolin, 4-[(R)-(1-Phenyl-ethyl)amino]-6-{[4-((R)-6-methyl-2-oxo-morpholin-4-yl)-1-oxo-2-buten-1-yl]amino}-7-methoxy-chinazolin, 4-[(3-Chlor-4-fluorphenyl)amino]-6-{[4-(morpholin-4-yl)-1-oxo-2-buten-1-yl]amino}-7-[(tetrahydrofuran-2-yl)methoxy]-chinazolin, 4-[(3-Ethinyl-phenyl)amino]-6-{[4-(5,5-dimethyl-2-oxo-morpholin-4-yl)-1-oxo-2-buten-1-yl]amino}-chinazolin, 4-[(3-Chlor-4-fluor-phenyl)amino]-6-{2-[4-(2-oxo-morpholin-4-yl)-piperidin-1-yl]-ethoxy}-7-methoxy-chinazolin, 4-[(3-Chlor-4-fluor-phenyl)amino]-6-(trans-4-amino-cyclohexan-1-yloxy)-7-methoxy-chinazolin, 4-[(3-Chlor-4-fluor-phenyl)amino]-6-(trans-4-methansulfonylamino-cyclohexan-1-yloxy)-7-methoxy-chinazolin, 4-[(3-Chlor-4-fluor-phenyl)amino]-6-(tetrahydropyran-3-yloxy)-7-methoxy-chinazolin, 4-[(3-Chlor-4-fluor-phenyl)amino]-6-{1-[(morpholin-4-yl)carbonyl]-piperidin-4-yloxy}-7-methoxy-chinazolin, 4-[(3-Chlor-4-fluor-phenyl)amino]-6-(piperidin-3-yloxy)-7-methoxy-chinazolin, 4-[(3-Chlor-4-fluor-phenyl)amino]-6-[1-(2-acetylamino-ethyl)-piperidin-4-yloxy]-7-methoxy-chinazolin, 4-[(3-Chlor-4-fluor-phenyl)amino]-6-(tetrahydropyran-4-yloxy)-7-ethoxy-chinazolin, 4-[(3-Chlor-4-fluor-phenyl)amino]-6-{trans-4-[(morpholin-4-yl)carbonylamino]-cyclohexan-1-yloxy}-7-methoxy-chinazolin, 4-[(3-Chlor-4-fluor-phenyl)amino]-6-{1-[(piperidin-1-yl)carbonyl]-piperidin-4-yloxy}-7-methoxy-chinazolin, 4-[(3-Chlor-4-fluor-phenyl)amino]-6-(cis-4-{N-[(morpholin-4-yl)carbonyl]-N-methyl-amino}-cyclohexan-1-yloxy)-7-methoxy-chinazolin, 4-[(3-Chlor-4-fluor-phenyl)amino]-6-(trans-4-ethansulfonylamino-cyclohexan-1-yloxy)-7-methoxy-chinazolin, 4-[(3-Chlor-4-fluor-phenyl)amino]-6-(1-methansulfonyl-piperidin-4-yloxy)-7-(2-methoxy-ethoxy)-chinazolin, 4-[(3-Chlor-4-fluorphenyl)amino]-6-[1-(2-methoxy-acetyl)-piperidin-4-yloxy]-7-(2-methoxy-ethoxy)-chinazolin, 4-[(3-Ethinyl-phenyl)amino]-6-(tetrahydropyran-4-yloxy]-7-methoxy-chinazolin, 4-[(3-Chlor-4-fluor-phenyl)amino]-6-(cis-4-{N-[(piperidin-1-yl)carbonyl]-N-methyl-amino}-cyclohexan-1-yloxy)-7-methoxy-chinazolin, 4-[(3-Chlor-4-fluorphenyl)amino]-6-{cis-4-[(morpholin-4-yl)carbonylamino]-cyclohexan-1-yloxy}-7-methoxy-chinazolin, 4-[(3-Chlor-4-fluor-phenyl)amino]-6-{1-[2-(2-oxopyrrolidin-1-yl)ethyl]-piperidin-4-yloxy}-7-methoxy-chinazolin, 4-[(3-Ethinyl-phenyl)amino]-6-(1-acetyl-piperidin-4-yloxy)-7-methoxy-chinazolin, 4-[(3-Ethinyl-phenyl)amino]-6-(1-methyl-piperidin-4-yloxy)-7-methoxy-chinazolin, 4-[(3-Ethinyl-phenyl)amino]-6-(1-methansulfonyl-piperidin-4-yloxy)-7-methoxy-chinazolin, 4-[(3-Chlor-4-fluorphenyl)amino]-6-(1-methyl-piperidin-4-yloxy)-7(2-methoxy-ethoxy)-chinazolin, 4-[(3-Ethinyl-phenyl)amino]-6-{1-[(morpholin-4-yl)carbonyl]-piperidin-4-yloxy}-7-methoxy-chinazolin, 4-[(3-Chlor-4-fluor-phenyl)amino]-6-{1-[(N-methyl-N-2-methoxyethyl-amino)carbonyl]-piperidin-4-yloxy}-7-methoxy-chinazolin, 4-[(3-Chlor-4-fluorphenyl)amino]-6-(1-ethyl-piperidin-4-yloxy)-7-methoxy-chinazolin, 4-[(3-Chlor-4-fluor-phenyl)amino]-6-[cis-4-(N-methansulfonyl-N-methyl-amino)-cyclohexan-1-yloxy]-7-methoxy-chinazolin, 4-[(3-Chlor-4-fluor-phenyl)amino]-6-[cis-4-(N-acetyl-N-methyl-amino)-cyclohexan-1-yloxy]-7-methoxy-chinazolin, 4-[(3-Chlor-4-fluorphenyl)amino]-6-(trans-4-methylamino-cyclohexan-1-yloxy)-7-methoxy-chinazolin, 4-[(3-Chlor-4-fluor-phenyl)amino]-6-[trans-4-(N-methansulfonyl-N-methyl-amino)-cyclohexan-1-yloxy]-7-methoxy-chinazolin, 4-[(3-Chlor-4-fluor-phenyl)amino]-6-(trans-4-dimethylamino-cyclohexan-1-yloxy)-7-methoxy-chinazolin, 4-[(3-Chlor-4-fluor-phenyl)amino]-6-(trans-4-{N-[(morpholin-4-yl)carbonyl]-N-methyl-amino}-cyclohexan-1-yloxy)-7-methoxy-chinazolin, 4-[(3-Chlor-4-fluor-phenyl)amino]-6-[2-(2,2-dimethyl-6-oxo-morpholin-4-yl)-ethoxy]-7-[(S)-(tetrahydrofuran-2-yl)methoxy]-chinazolin, 4-[(3-Chlor-4-fluor-phenyl)amino]-6-(1-methansulfonyl-piperidin-4-yloxy)-7-methoxy-chinazolin, 4-[(3-Chlor-4-fluor-phenyl)amino]-6-(1-cyano-piperidin-4-yloxy)-7-methoxy-chinazolin, und 4-[(3-Chlor-4-fluor-phenyl)amino]-6-{1-[(2-methoxyethyl)carbonyl]-piperidin-4-yloxy}-7-methoxy-chinazolin, gegebenenfalls in Form ihrer Racemate, Enantiomere oder Diastereomere, gegebenenfalls in Form ihrer pharmakologisch verträglichen Säureadditionssalze, ihrer Solvate und/oder Hydrate.

Für die Inhalation kommen Arzneimittel mit den o.g. Wirkstoffen in Betracht, sowie deren Salze, Ester sowie die Kombination dieser Wirkstoffe, Salze und Ester.

Die Inhalationspulver werden nach den aus dem Stand der Technik bekannten Verfahren, insbesondere jedoch durch Verfahren der Sprühtrocknung, hergestellt.

Die in die erfindungsgemäßen Blister eingesetzten Inhalationspulver enthalten neben dem Wirkstoff bevorzugt wenigstens einen Hilfsstoff. Dieser kann aus einer bezüglich der mittleren Teilchengröße der Hilfsstoffpartikel einheitlichen Hilfsstofffraktion (beispielsweise 15-80 Mikrometer) bestehen oder gegebenenfalls ein Gemisch von gröberem Hilfsstoff mit einer mittleren Teilchengröße von 15 bis 80 Mikrometer und feinerem Hilfsstoff mit einer mittleren Teilchengröße von 1 bis 9 Mikrometer darstellen. Werden Hilfsstoffgemische aus gröberen und feineren Hilfsstoffanteilen eingesetzt, beträgt der Anteil von feinerem Hilfsstoff an der Gesamthilfsstoffmenge vorzugsweise 1 bis 20%.

Als physiologisch unbedenkliche Hilfsstoffe seien beispielsweise genannt Monosaccharide (z.B. Glucose oder Arabinose), Disaccharide (z.B. Lactose, Saccharose, Maltose), Oligo- und Polysaccharide (z.B. Dextrane), Polyalkohole (z.B. Sorbit, Mannit, Xylit), Salze (z.B. Natriumchlorid, Calciumcarbonat) oder Mischungen dieser Hilfsstoffe miteinander. Bevorzugt gelangen Mono- oder Disaccharide zur Anwendung, wobei die Verwendung von Lactose oder Glucose, insbesondere, aber nicht ausschließlich in Form ihrer Hydrate, bevorzugt ist. Als besonders bevorzugt im Sinne der Erfindung gelangt Lactose, höchst bevorzugt Lactosemonohydrat als Hilfsstoff zur Anwendung.

Die bisher beschriebenen Blister stellen in der Regel Primärverpackungen dar, die in Form einer zylinderartigen Scheibe oder als streifenförmiger, elastischer Blister vorliegen können. Wie nachfolgend noch beschrieben, können die Blister zur Freisetzung der Inhalationsformulierung entweder mit einem Anstechelement geöffnet oder durch Abziehen der Materialbahnen geöffnet werden. Die Blister können mit einer oder mehreren Einzeldosen bestückt sein. Diese können zusätzlich mit einer Polymer und/oder Aluminium-Schicht bestückt sein. Auch kann der einsatzfähige Inhalator in einen Beutel aus einem Polymer oder aus einer Aluminiumfolie eingeschweißt aufbewahrt werden. Die Verwendung eines Sekundärpackmittels hat verschiedene Vorteile. Zum einen wird die Funktionalität der erfindungsgemäßen Primärverpackungen, insbesondere die Funktionalität des Entwässerungsmittels dadurch zeitlich verlängert, dass die zeitlich begrenzte Barrierewirkung gegenüber Wasser erst nach Öffnen der Sekundärpackung zum Tragen kommt, was zusätzliche Sicherheit der Produktqualität ermöglicht. Zum anderen kann der Lagerzeitfeuchteschutz und Feuchteschutz bei Offenlagerung in der ungeöffneten Sekundärverpackung dadurch verlängert werden, dass in die Sekundärverpackung eindringende Feuchte vom entwässernden Kunststoff aufgenommen wird.

Die erfindungsgemäße Blisterscheibe kann eine zylinderartige Scheibe von bis zu 5 mm Höhe und einem Durchmesser von bis zu 15 cm sein. In der Scheibe sind senkrecht zur Scheibenebene Mulden oder Löcher ausgebildet (Kavitäten). Die Mulden oder Löcher sind bevorzugt am äußeren Rand der Scheibe ausgebildet und können durch eine oder mehrere Folien verschlossen sein. In den Mulden oder Löchern befindet sich die Inhalationsformulierung. Der Scheibenkörper besteht dabei aus dem erfindungsgemäß eingesetzten Material. Eine derartige Scheibe kann beispielsweise in einem Inhalator gemäß der DE 3348370 oder der DE 3336486 eingesetzt werden. Ein solcher Inhalator weist ein Gehäuse auf, in dem sich der scheibenförmige, runde Blister mit abgepackten Medikamententaschen befindet. Der Inhalator weist u.a. einen Stift auf, der so angeordnet ist, dass er jeweils eine Medikamententasche öffnen kann, so dass das Medikament in die Kammer freigesetzt wird und über ein Mundstück eingeatmet werden kann.

In einer anderen Ausführungsform ist der Behälter ein streifenförmiger elastischer Blister mit Medikamentaschen, wie er in der DE 4106379 beschrieben wird. Der Blister besteht aus wenigstens zwei voneinander abziehbaren Materialbahnen, die wenigstens zwei Behälter definieren, in dem sich jeweils das Medikament befindet. Das Material kann dabei das eingangs beschriebene für die Erfindung geeignete Material sein. In Varianten dieser Ausbildungsform handelt es sich bei der Primärverpackung um einen vergleichbaren Blister bei dem eine Aluminiumfolie und eine Folie gemäß dem erfindungsgemäßen Material laminiert sind. Dabei kann die mit der Inhalationsformulierung in Kontakt kommende Seite die Aluminiumseite sein. In wieder anderen Varianten handelt es sich um Laminate der Schichtenfolge Aluminium, Polymer mit Entwässerungsmittel, Aluminium. Der dazugehörige Inhalator ist versehen mit einer Einrichtung, die zum Öffnen dieses Blisters die beiden Materialbahnen an einer Öffnungsstation voneinander abzieht. Über ein Auslassteil, etwa ein Mundstück, das mit dem geöffneten Behälter verbunden ist, kann der Benutzer das pulverförmige Medikament aus dem geöffneten Behälter inhalieren. Dabei kann eine der Materialbahnen auch eine Trägerbahn mit mehreren Taschen sein und die andere Materialbahn eine Abdeckbahn. Jede Tasche und der angrenzende Bereich der Abdeckbahn bilden dann einen Behälter. An der Öffnungsstation kann eine Antriebseinrichtung vorgesehen sein, die die Trägerbahn und die Abdeckbahn voneinander abzieht. Diese Antriebseinrichtung besteht z.B. aus zwei Antriebsrädern (z.B. Zahnräder), welche die Abdeckbahn in Antriebseingriff zwischen sich halten. Auch in diesem Fall definiert jeder einzelne Blister ein Art Vorratskammer im Inhalator, die über einen Luftkanal mit dem Mundstück verbunden ist.

Alternativ, auberbolb der Erfindung, können den Inhalationsformulierungen Körper aus dem eingangs beschriebenen Polymermaterial mit entwässernden Eigenschaften beigemengt werden. Die Größe dieser Formkörper ist dabei größer als das größte Partikel der Arzneimittelformulierung, so dass die Körper durch Sieben von den Formulierungspartikeln getrennt werden können. Damit diese Körper nicht mit inhaliert werden, müssen sie während der Inhalation ausgesiebt werden. Dafür können die Mundstücke oder andere Bereiche der Inhalatoren mit einem entsprechenden Sieb versehen werden. In wieder einer anderen Alternative sind die Körper so ausgebildet, dass sie zu schwer sind, um mit den Arzneimittelpartikeln beim Inhalationsvorgang mitgerissen zu werden. Der entwässernde Kunststoff kann in Form von einem oder mehreren Körper(n) in die Kapsel eingebracht werden. Bei der Verwendung von mehr als einem Körper können jeweils gleiche oder verschiedene Geometrien in einer Kapsel Verwendung finden. Bevorzugt sind eine kugelförmige oder rotationsellipsoide Geometrien.
In diesem Fall muss das Basisteil des Blisters nicht aus dem die Entwässerungsmittel enthaltendem Polymer gefertigt sein. In einem Beispiel mit einem frei beweglichen Formkörper aus einem ein entwässerungsmittel enthaltendem Material kann die Formulierung und der Formkörper in einer zweiteiligen, teleskopartigen Kapsel vorhanden sein, die in entsprechende Inhalatoren eingesetzt werden kann. Derartige Kapseln sind beispielsweise in der EP 1100474 beschreiben, auf die hiermit in vollem Umfang Bezug genommen wird. Bevorzugte Kapselgröße ist 3. Ein geeigneter Inhalator ist beispielsweise ein Gerät der Marke HandiHaler^{®}, wie er z.B. in der EP 1342483 offenbart wird. Ein Form dieses Aspekts betrifft ein Ensemble aus einem Inhalator für die Inhalation pulverförmiger Arzneimittel und einer zweiteiligen Kapsel, die den erfindungsgemäßen Formkörper enthält, wobei der Inhalator gekennzeichnet ist durch a) ein nach oben hin offenes, becherförmiges Unterteil, welches in der Ummantelung zwei gegenüber liegende Fenster aufweist und am Rand der Öffnung ein erstes Scharnierelement hat, b) eine Platte, welches die Öffnung des Unterteils bedeckt und ein zweites Scharnierelement aufweist, c) eine Inhalationskammer zum Aufnehmen der Kapsel, die senkrecht zur Plattenebene an der zum Unterteil weisenden Seite der Platte ausgebildet ist und an der ein gegen eine Feder beweglicher Kopf vorgesehen ist, wobei der Kopf mit zwei geschliffenen Nadeln versehen ist, d) ein Oberteil mit einem Mundrohr und einem dritten Scharnierelement, sowie e) einen Deckel, der ein viertes Scharnierelement aufweist, wobei die Scharnierelemente eins des Unterteils, zwei der Platte, drei des Oberteils und vier des Deckels miteinander verbunden sind.

### Beispiel 1

Die Verlängerung des Schutzes von Blister-Packungen vor eindringender Feuchtigkeit bei Langzeit- und Offenlagerung bei 40°C und 75% r.F. (Behältnisse gefüllt mit Inhalationspulver aus einer Tiotropium/Lactose-Mischung):

Mit Hilfe geeigneter Messverfahren wurde ermittelt, dass Blister-Packungen, bei Langzeit- und Offenlagerung bei 40°C und 75% r.F. Wasser (r.F. = relative Feuchte) mit einer Rate von 0.1 bis 100 mg pro Jahr durch den Blister durchlassen. Die aufgenommene Wassermenge hängt dabei sehr stark von den verwendeten Blistermaterialien und der Dichtigkeit der Versiegelung ab. Die nachfolgende Tabelle enthält eine Übersicht, die die Verlängerung des Feuchteschutzes in Abhängigkeit von der Blister-Leckrate in mg/Jahr und vom Masseanteil Molekularsieb in Gew.-% zeigt.

| **Gesamtmasse MS* im Blister** | **Wasseraufnahme-kapazität MS*** | **Blister Leckrate pro Jahr** | **Verlängerung des Feuchteschutzes** |
|---|---|---|---|
| 10 mg | 1.5 mg | 0.1 mg | + 15.0 Jahre |
| 39 mg | 5.9 mg | 1 mg | + 5.9 Jahre |
| 65 mg | 9.8 mg | 5 mg | + 2.0 Jahre |
| 117 mg | 17.6 mg | 10 mg | + 1.8 Jahre |
| 78 mg | 11.7 mg | 10 mg | + 1.2 Jahre |
| 130 mg | 19.5 mg | 20 mg | + 1.0 Jahre |
| 260 mg | 30.0 mg | 50 mg | + 0.8 Jahre |
| 195 mg | 29.3 mg | 50 mg | + 0.6 Jahre |
| 390 mg | 58.5 mg | 100 mg | + 0.6 Jahre |

| | | | |
|---|---|---|---|
| * MS: Molekularsieb | | | |

Die Werte in der Spalte "Verlängerung des Feuchteschutzes" zeigen, dass bei niedriger Blister-Leckrate von 1 mg/Jahr oder weniger eine Verlängerung des Schutzes um 6 Jahre oder mehr bei Umgebungsbedingungen von 40°C und 75% r.F. erreicht werden kann. Bei hohen Blister-Leckraten von 100 mg Wasser pro Jahr erreicht man bei gleichen Umgebungsbedingungen eine Verlängerung des Feuchteschutzes um 7 Monate. Dieses Beispiel ist auf die Situation der Anbruchstabilität der Blisterpackung direkt übertragbar, wenn sich die beschriebene Blisterpackung für die Langzeitlagerung in einer hermetisch dichten Umverpackung befindet.

## Patentansprüche

1. Blister, der integraler Bestandteil eines gebrauchsfertigen Pulverinhalators ist, ausgewählt aus der Gruppe der Blisterstreifen oder der Blisterscheiben, bestehend aus einem Basiselement mit wenigstens zwei nach wenigstens einer Seite hin offenen Kavitäten, die durch einen Steg voneinander getrennt sind und die eine feuchtigkeitsempfindliche Inhalationsformulierungen enthalten, und die wenigstens eine Öffnung der Kavitäten versiegelt ist, wobei die Wandung der Kavität aus wenigstens zwei Schichten, einer inneren und wenigstens einer darüber liegenden äußeren Schicht besteht und wobei die innere Schicht die unmittelbare Wandung der Kavität bildet und in Kontakt mit der Inhalationsformulierung steht, dadurch charakterisiert, dass die innere Schicht aus einer Polymerzusammensetzung ohne Entwässerungsmittel besteht und die umgebende zweite Schicht, die die Inhalationsformulierung nicht berührt, aus einer Polymerzusammensetzung mit einem entwässerndem Material besteht.

2. Blister nach Anspruch 1, **dadurch gekennzeichnet, dass** es sich bei dem entwässernden Material um eine Polymerzusammensetzung bestehend aus ein oder mehreren verschiedenartigen Polymeren und/oder ein oder mehreren verschiedenartigen Entwässerungsmitteln handelt.

3. Blister nach einem der vorangegangenen Ansprüche, **dadurch gekennzeichnet, dass** das entwässernde Material Kieselgele, Aktivkohle, Zeolithe, Aluminiumoxid, Magnesiumsulfat , Molekularsiebe umfasst.

4. Blister nach einem der vorangegangenen Ansprüche, **dadurch gekennzeichnet, dass** es sich bei den verwendeten Polymeren um thermoplastische Polymere handelt wie z.B. Polystyrole, Polyolefine, Polyamide, Polyvinylchloride oder Polyurethane, Polyethylen, Polycarbonat, Polyester, Polypropylen oder Polyethylenterephthalat.

5. Blister nach einem der vorangegangenen Ansprüche, **dadurch gekennzeichnet, dass** die Polymerzusammensetzung auch ein oder mehrere Elastomere enthält, bevorzugt ausgewählt aus der Gruppe der Styrol-Butadien-Kautschuke, Styrol-Ethylen-Butylen-Styrol-Copolymere, Butyl-Kautschuke, Ethylen-Propylen-Kautschuke, Ethylen-Propylen-Dien-Kautschuke, Ethylen-Vinylacetat-Copolymere, Ethylen-Acrylat-Copolymere, Acrylnitril-Butadien-Copolymere, Polynorbonene, Polyiisoprene, Polychloroprene und Polybutadiene.

6. Blister nach einem der vorangegangenen Ansprüche, **dadurch gekennzeichnet, dass** die Polymerzusammensetzung Weichmacher, Stabilisatoren, Farbstoffe oder Pigmente enthält.

7. Blister nach einem der vorangegangenen Ansprüche, **dadurch gekennzeichnet, dass** die Inhalationsformulierung Tiotropiumbromid-Monohydrat enthält.

8. Blister nach einem der vorangegangenen Ansprüche, **dadurch gekennzeichnet, dass** der Blister als zusätzliches Element einen Körper enthält, welcher aus entwässerndem Material besteht.

9. Blister nach Anspruch 8, **dadurch gekennzeichnet, dass** das entwässernde Material des zusätzlichen Elementes ein Material gemäß Anspruch 2 ist.

10. Blister nach einem der vorangegangenen Ansprüche, **dadurch gekennzeichnet, dass** der Blister eine Blisterscheibe ist.

11. Blister nach einem der vorangegangenen Ansprüche, **dadurch gekennzeichnet, dass** der Blister ein scheibenförmiger Blister von bis zu 5 mm Höhe und einem Durchmesser von bis zu 15 cm ist, an dessen äußerem Rand senkrecht zur Scheibenebene Mulden oder Löcher ausgebildet, deren Öffnung durch eine oder mehrere Folien verschlossen sind.

12. Blister nach einem der vorangegangenen Ansprüche, **dadurch gekennzeichnet, dass** der Blister ein anstechbarer Blister ist.

13. Blister nach einem der vorangegangenen Ansprüche, **dadurch gekennzeichnet**, der Blister ein streifenförmiger elastischer Blister mit Medikamentaschen ist, der aus wenigstens zwei voneinander abziehbaren Materialbahnen besteht.

14. Inhalator enthaltend ein Blister nach einem der Ansprüche 1 bis 13, wobei der Inhalator eine oder mehrere Einzeldosen enthalten kann.

## Claims

1. Blister which is an integral component of a ready-to-use powder inhaler, selected from the group comprising blister strips or blister discs, comprising a base element with at least two cavities that are open toward at least one side and are separated from each other by a web and which contain a moisture-sensitive inhalable formulation, and the at least one opening of the cavities is sealed, the wall of the cavity consisting of at least two layers, an inner layer and at least one outer layer located thereon, and the inner layer forming the immediate wall of the cavity and being in contact with the inhalable formulation, **characterised in that** the inner layer consists of a polymer composition with no dehydrating agents and the surrounding second layer which does not come into contact with the inhalable formulation consists of a polymer composition with a dehydrating material.

2. Blister according to Claim 1, **characterised in that** the dehydrating material is a polymer composition comprising one or more different polymer(s) and/or one or more different dehydrating agents.

3. Blister according to either of the preceding claims, **characterised in that** the dehydrating material comprises silica gels, activated carbon, zeolites, aluminium oxide, magnesium sulphate and molecular sieves.

4. Blister according to any one of the preceding claims, **characterised in that** the polymers used are thermoplastic polymers such as polystyrenes, polyolefins, polyamides, polyvinylchlorides or polyurethanes, polyethylene, polycarbonate, polyester, polypropylene or polyethylene terephthalate.

5. Blister according to any one of the preceding claims, **characterised in that** the polymer composition also contains one or more elastomers, preferably selected from the group comprising styrene butadiene rubbers, styrene ethylene butylene styrene copolymers, butyl rubbers, ethylene propylene rubbers, ethylene propylene diene rubbers, ethylene vinylacetate copolymers, ethylene acrylate copolymers, acrylonitrile butadiene copolymers, polynorbonenes, polyisoprenes, polychloroprenes and polybutadienes.

6. Blister according to any one of the preceding claims, **characterised in that** the polymer composition contains plasticisers, stabilisers, dyes or pigments.

7. Blister according to any one of the preceding claims, **characterised in that** the inhalable formulation contains tiotropium bromide monohydrate.

8. Blister according to any one of the preceding claims, **characterised in that**, as an additional element, the blister contains a body which consists of dehydrating material.

9. Blister according to Claim 8, **characterised in that** the dehydrating material of the additional element is a material according to Claim 2.

10. Blister according to any one of the preceding claims, **characterised in that** the blister is a blister disc.

11. Blister according to any one of the preceding claims, **characterised in that** the blister is a disc-shaped blister up to 5 mm high and with a diameter of up to 15 cm, on the outer edge of which and perpendicular to the disc plane troughs or holes are formed, the openings of which are closed by one or more film(s).

12. Blister according to any one of the preceding claims, **characterised in that** the blister is a pierceable blister.

13. Blister according to any one of the preceding claims, **characterised in that** the blister is a strip-shaped elastic blister with medicament pouches and comprises at least two strips of material that can be pulled away from each other.

14. Inhaler containing a blister according to any one of Claims 1 to 13, wherein the inhaler may contain one or more single dose(s).

## Revendications

1. Blister qui fait partie intégrante d'un inhalateur de poudre prêt à l'emploi, choisi dans le groupe des blisters en bande ou des blisters en disque, consistant en un élément de base comportant au moins deux cavités ouvertes vers au moins un côté, qui sont séparées l'une de l'autre par un cloisonnement et qui contiennent une formulation pour inhalation sensible à l'humidité et au moins une ouverture des cavités étant scellée, dans lequel la paroi de la cavité est constituée d'au moins deux couches, une couche interne et au moins une couche externe se trouvant sur celle-ci, et dans lequel la couche interne forme la paroi directe de la cavité et est en contact avec la formulation pour inhalation, **caractérisé en ce que** la couche interne consiste en une composition de polymère sans agent déshydratant et la deuxième couche l'entourant qui ne touche pas la formulation pour inhalation consiste en une composition de polymère avec un matériau déshydratant.

2. Blister selon la revendication 1, **caractérisé en ce que** le matériau déshydratant est une composition de polymère consistant en un ou plusieurs polymères de différentes sortes et/ou un ou plusieurs agents déshydratants de différentes sortes.

3. Blister selon l'une des revendications précédentes, **caractérisé en ce que** le matériau déshydratant comprend des gels de silice, des charbons actifs, des zéolithes, de l'oxyde d'aluminium, du sulfate de magnésium, des tamis moléculaires.

4. Blister selon l'une des revendications précédentes, **caractérisé en ce que** les polymères utilisés sont des polymères thermoplastiques tels que par exemple, des polystyrènes, des polyoléfines, des polyamides, des chlorures de poly-vinyle ou des polyuréthanes, du polyéthylène, du polycarbonate, du polyester, du polypropylène ou du polyéthylène téréphtalate.

5. Blister selon l'une des revendications précédentes, **caractérisé en ce que** la composition de polymère contient également un ou plusieurs élastomères, choisis de préférence dans le groupe des caoutchoucs de styrène-butadiène, des copolymères de styrène-éthylène-butylène-styrène, des caoutchoucs butyle, des caoutchoucs d'éthylène-propylène, des caoutchoucs d'éthylène-propylène-diène, des copolymères d'éthylène-acétate de vinyle, des copolymères d'éthylène-acrylate, des copolymères d'acrylonitrile-butadiène, des poly-norbornènes, des polyisoprènes, des polychloro-prènes et des polybutadiènes.

6. Blister selon l'une des revendications précédentes, **caractérisé en ce que** la composition de polymère contient des plastifiants, des stabilisants, des colorants ou des pigments.

7. Blister selon l'une des revendications précédentes, **caractérisé en ce que** la formulation pour inhalation contient du bromure de tiotropium monohydrate.

8. Blister selon l'une des revendications précédentes, **caractérisé en ce que** le blister contient, comme élément supplémentaire, un corps qui consiste en un matériau déshydraté.

9. Blister selon la revendication 8, **caractérisé en ce que** le matériau déshydraté de l'élément supplémentaire est un matériau selon la revendication 2.

10. Blister selon l'une des revendications précédentes, **caractérisé en ce que** le blister est un blister en disque.

11. Bister selon l'une des revendications précédentes, **caractérisé en ce que** le blister est un blister en forme de disque de jusqu'à 5 mm de hauteur et d'un diamètre de jusqu'à 15 cm, au bord extérieur duquel sont formés, verticalement par rapport au plan du disque, des cuvettes ou des trous dont l'ouverture est fermée par une ou plusieurs feuilles.

12. Blister selon l'une des revendications précédentes, **caractérisé en ce que** le blister est un blister perforable.

13. Blister selon l'une des revendications précédentes, **caractérisé en ce que** le blister est un blister élastique en forme de bandes comportant des poches de médicaments qui consistent en au moins deux bandes de matériaux séparables l'une de l'autre.

14. Inhalateur contenant un blister selon l'une des revendications 1 à 13, l'inhalateur pouvant contenir une ou plusieurs doses individuelles.
